# EUROPÄISCHE PATENTSCHRIFT

(11) **EP 2 967 970 B1**
(45) Veröffentlichungstag und Bekanntmachung des Hinweises auf die Patenterteilung: **23.08.2017**
(21) Anmeldenummer: 14707933.9
(22) Anmeldetag: 12.02.2014
(51) Int. Cl.: A61F 5/56

(54) **AUFBISSSCHIENE**
BITE SPLINT
APPAREIL DENTAIRE

(30) Priorität: 12.03.2013 DE 202013002334 U
(43) Veröffentlichungstag der Anmeldung: 20.01.2016
(73) Patentinhaber: Yildiz, Fahri, 50933 Köln (DE)
(72) Erfinder: Toussaint, Winfried, 64646 Heppenheim (DE)
(74) Vertreter: Kompter, Hans-Michael
(86) Internationale Anmeldenummer: PCT/DE2014/000052
(87) Internationale Veröffentlichungsnummer: WO 2014/139492

(56) Entgegenhaltungen:
- EP-A2- 1 203 570
- WO-A1-2004/110570
- US-A- 4 568 280
- US-A- 5 117 816
- US-A- 5 931 164
- US-A1- 2012 285 473

## Beschreibung

### 1. TECHNISCHES GEBIET

Die Erfindung betrifft eine Aufbissschiene aus thermoplastischen Material zur Verhinderung von Schnarchen und von obstruktiver Schlafapnoe, umfassend eine Unter- und eine Oberseite, die jeweils einen zum Unter- bzw. Oberkiefer geöffneten, Formgebenden Einbisskanal für die Zähne bilden, und das thermoplastische Material, welches zahnspangenartig dem menschlichen Ober- und Unterkiefer nachformbar ist.

### 2. STAND DER TECHNIK

Schnarchen kann ein Symptom sein für nächtliche Atemstillstände, die schwerwiegende gesundheitliche Komplikationen, wie z.B. Herz-Kreislauferkrankungen nach sich ziehen können. Durch das US Patent US 5,462,066 ist eine derartige zahnspangenartige Aufbissschiene zur Verhinderung des Schnarchens bekannt geworden, welche dazu dient, den Unterkiefer geringfügig nach vorne zu verschieben, weil in dieser Stellung des Unterkiefers die Atemwege weiter geöffnet werden, so dass der Patient freier atmen kann, ohne zu schnarchen.

Das US Patent US 5,313,960 schlägt eine zweiteilige Aufbissschiene mit einem harten und einem anpassbaren weichen Anteil vor, deren obere und untere Hälfte zur Anpassung an das Gebiss des Verwenders mit einem gewissen Vorschub des oberen Teils auf einem Halter befestigt werden, der nach Anpassung entfernt wird und die beiden Teile wieder direkt miteinander verbunden werden. Zur besseren Anpassung kann der Zahnkanal Erhöhungen aufweisen.

Auch bei der Unterkieferschiene des US Patentes US 5,720,302 wird vorgeschlagen, dass die innere Oberfläche eine Vielzahl von räumlich getrennten Gebieten zur Zahnaufnahme aufweist, welche durch Trennrippen voneinander getrennt sind.

Die bekannte einteilige Aufbissschiene in Form eines zahnspangenartigen Mundstücks besteht aus einem thermoplastischen Material mit zwei Bissrillen, welches mit Erwärmung formbar wird. Der Patient nimmt das erwärmte, noch nicht angepasste Mundstück in den Mund, um dann in den formbaren Kunststoff die Zähne des Unter- sowie des Oberkiefers in die entsprechende untere sowie obere Bissrille hineinzudrücken und durch Aufbeißen auf die Bissplatten der Bissrillen anzupassen. Dabei kühlt der Kunststoff ab und gewinnt seine feste Elastizität zurück, wonach nunmehr das Mundstück an die oberen und unteren Zahnreihen des Patienten angepasst ist. Beim Anpassungsvorgang muss darauf geachtet werden, den Unterkiefer etwas nach vorne zu verschieben.

Diese Aufbissschiene besitzt den Nachteil, dass dieselbe im Bereich der übereinander liegenden Bissrillen zu dünn ausgeführt ist, so dass ein Durchbeißen der Bissplatte nach häufigem Benutzen möglich ist. Des Weiteren besitzt die bekannte Aufbissschiene keine Notatemluftöffnung, so dass bei Schlafapnoe in Extremfällen sogar eine lebensgefährliche Situation entstehen kann.

In dem US Patent US 4,568,280 wird eine Aufbissschiene vorgeschlagen, bei der es sich jedoch nicht um eine Unterkieferprotrussionsschiene zur Verhinderung von Schnarchen und von obstruktiver Schlafapnoe handelt, sondern um eine Schiene zur Korrektur von Fehlstellungen der Kiefer zueinander wie Retrogranathismus. Der Einbisskanal dieser Schiene weist Rippen auf.

Bei der in dem US Patent US 5,931,164 offenbarten Vorrichtung handelt es sich um einen Mundschutz und nicht um eine Aufbissschiene zur Verhinderung des Schnarchens oder von obstruktiver Schlafapnoe. Weiterhin weist sie lediglich einen zum Oberkiefer geöffneten Einbisskanal auf.

In der europäischen Patentanmeldung EP 1 203 570 A2, die als nächster Stand der Technik angesehen wird, wird eine einteilige Aufbissschiene vorgeschlagen, wobei das thermoplastische Kunststoffgemisch aus einer Mischung von zwei thermoplastischen Co-polymeren im Verhältnis von 1**:**1 oder mehr oder weniger 1:1 besteht, welche beide ihrerseits aus Polyethylen und Polyvinylacetat bestehen, und der Gehalt an Vinylacetat zwischen 25 bis 33 Gewichtsprozent beträgt.

Die bisher bekannten einteiligen thermoplastischen Aufbisschienen neigen dazu, dass die Haftung (Retention) der Zähne nach längerem Gebrauch nachlässt oder bei nicht fachgerechter Anpassung unzureichend ist

Der Erfindung liegt die Aufgabe zugrunde, eine thermoplastische Aufbissschiene zur Verhinderung von Schnarchen sowie von obstruktiver Schlafapnoe zu schaffen, welche gegenüber dem Stand der Technik verbessert ist und insbesondere von der noch nicht angepassten Form ausgehend eine einfache höchst individuelle Anpassung an das Desmodont des Patienten ermöglicht.

Eine weitere Aufgabe der vorliegenden Erfindung war es, die Haftung der Zähne (Retention) zu verbessern.

Es wurde nun überraschenderweise gefunden, dass die Haftung der Zähne, insbesondere nach längerem Gebrauch der Aufbissschiene deutlich verbessert werden kann, wenn die innere Oberfläche des Formgebenden und biegesteifen Einbisskanals, eine Struktur aufweist, welche die Lage der Zähne in dem Einbisskanal definiert und beim Einbiss stabilisiert.

### KURZE ZUSAMMENFASSUNG DER ERFINDUNG

Gegenstand der Erfindung ist somit eine Aufbissschiene (1) aus thermoplastischen Material zur Verhinderung von Schnarchen und von obstruktiver Schlafapnoe, welche es erlaubt, den Unterkiefer gegenüber dem Oberkiefer in eine leicht nach vorne gelagerte Position zu verschieben, umfassend eine Unter- (2) und eine Oberseite (3), die jeweils einen zum Unter- bzw. Oberkiefer geöffneten, Formgebenden Einbisskanal (4, 5) für die Zähne bilden, und das thermoplastische Material, das zahnspangenartig dem menschlichen Ober- und Unterkiefer nachformbar ist, wobei die innere Oberfläche der beiden Einbisskanäle eine Struktur aufweist, welche die Lage der Zähne in den Einbisskanälen definiert und deren Haftung (Retention) in dem thermoplastischen Material optimiert. Diese Struktur der Einbisskanäle wird dadurch geformt, dass ihr innerer Boden (12) im Bereich der Eck- und Backenzähne eine oder mehrere flache Erhöhungen (13) mit einem im Wesentlichen kreisförmigen Bodenumfang aufweist und ihre inneren Seitenwände eine oder mehrere Verengungen in Form von vertikalen Stegen (14, 15) aufweisen.

### KURZE BESCHREIBUNG DER ABBILDUNGEN

Figur 1 zeigt eine perspektivische Ansicht der Oberseite von hinten einer Ausführungsform der erfindungsgemäßen Aufbissschiene;
Figur 2 zeigt eine perspektivische Ansicht der Unterseite der in Fig. 1 gezeigten Ausführungsform, die um 180 ° gedreht wurde, von hinten;
Figur 3 zeigt eine Ansicht einer Ausführungsform der erfindungsgemäßen Aufbissschiene von hinten;
Figur 4 zeigt eine photographische Aufnahme einer weiteren Ausführungsform der erfindungsgemäßen Aufbissschiene seitlich von vorne;

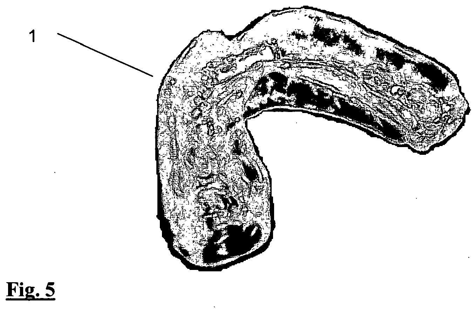

Figur 5 zeigt eine photographische Aufnahme einer weiteren Ausführungsform der erfindungsgemäßen Aufbissschiene mit thermoplastischem Füllmaterial nach Anpassung seitlich von vorne.

### DETAILLIERTE BESCHREIBUNG DER ERFINDUNG

Die erfindungsgemäße Aufbissschiene lässt sich aus bekannten Materialien universell massengefertigt im Spritzgussverfahren herstellen.

Der Begriff "Aufbissschiene" wie er vor- und nachstehend verwendet wird bezeichnet eine dentale Vorrichtung, welche es erlaubt, den Unterkiefer gegenüber dem Oberkiefer in eine leicht nach vorne gelagerte Position zu verschieben und dadurch den Querschnitt der oberen Atemwege zu vergrößern. Dies führt zu einer Reduktion des Schnarchens und von Atemaussetzern infolge einer obstruktiven Schlafapnoe. Die Aufbissschiene kann ein- oder zweiteilig sein, wobei bei einer zweiteiligen ein Verbindungselement das Ober- und Unterteil mit einander lösbar verbindet. Im vorliegenden Fall sind einteilige Aufbissschienen bevorzugt, wobei der Boden der Unterkiefer und Oberkieferschale eine gemeinsame Bissplatte bilden.

Der Begriff "thermoplastisches Material" wie er vor- und nachstehend verwendet wird bezeichnet ein Material, das sich in der Wärme, vorzugsweise unterhalb von 60 °C, vorzugsweise zwischen 40 und 50 °C plastisch verformen lässt und sich eng an eine vorgegebene Form anschmiegt und danach beim Abkühlen diese besagte Form beibehält. Geeignete Materialien sind Polymere und Co-polymere oder Gemische davon aus der Gruppe der Polyethylene (PE), Polyvinylacetate (PVA), Acrylate und Methacrylate, bevorzugt Co-polymere aus PE und PVA, wie sie zum Beispiel unter der Marke Elvax® der Firma DuPont erhältlich sind. Bevorzugt sind solche PE-PVA Co-polymere, deren PVA Gehalt von 20 bis 40, insbesondere 25 bis 35 % beträgt

Die vor- und nachstehend verwendeten Angaben hinsichtlich der Geometrie oder räumlichen Anordnung der Aufbissschiene oder von Teilen davon orientieren sich an den Gegebenheiten der Aufbissschiene bei ihrer sinngemäßen Verwendung, d.h. nach Einsetzen derselben auf die obere und untere Zahnreihe der betroffenen Person: "in Längsrichtung" oder bedeutet in Richtung der Mundöffnung oder der Wangen ("anterior" oder "bukkal") oder des Rachens ("posterior" oder "lingual"); "nach vorne" (anterior) bedeutet in Richtung der Mundöffnung; "mittig" bedeutet im Bereich der Schneidezähne; "hinten" bedeutet im Bereich der Backenzähne.

Vorteilhafte Ausgestaltungen der Erfindung sind solche Aufbissschienen, wobei
(a) der innere Boden der beiden Einbisskanäle jeweils 3 bis 12 flache Erhöhungen, vorzugsweise mit im Wesentlichen kreisförmigen Bodenumfang aufweist.
(b) die inneren Seitenwände der beiden Einbisskanäle an der bukkalen Seitenwand 8 bis 15 und an der lingualen Seitenwand 6 bis 13 Verengungen (7) in Form von vertikalen Stegen aufweisen.
(c) der innere Boden des oberen Einbisskanals auf beiden Seiten jeweils im Bereich der Backenzähne 4 flache Erhöhungen aufweist und der innere Boden des unteren Einbisskanals auf beiden Seiten jeweils im Bereich der Backenzähne 5 flache Erhöhungen aufweist. Diese Erhöhungen sind vorzugsweise kreisförmig und ragen aus der Bissplatte um 1 bis 4 mm, insbesondere um etwa 2 mm heraus.
(d) die inneren Seitenwände der beiden Einbisskanäle jeweils auf der bukkalen und lingualen Seite im Bereich der Backenzähne 2 bis 5, insbesondere 4 Verengungen, und/oder auf der bukkalen und lingualen Seite im Bereich der Eck und Schneidezähne 2 bis 4, insbesondere 3, Verengungen aufweisen.
(e) jede der vorderen flachen Erhöhungen des inneren Bodens von 4 Verengungen der inneren Seitenwände flankiert ist.
(f) die Verengungen der inneren Seitenwände im getragen Zustand jeweils in den Zahnzwischenräumen liegen und die flachen Erhöhungen des inneren Bodens jeweils im Bereich der Zahnkronen liegen.
(g) der Boden im hinteren Bereich der Einbisskanal über die Seitenwände vorzugsweise um 1,5 bis 4,5 mm, insbesondere um etwa 3,5 mm hinausragt.

Ganz besonders bevorzugt sind Aufbissschienen, wobei der Einbisskanal der Oberseite insgesamt 22 vertikale Stege und 8 kreisförmige Erhebungen aufweist, und/oder der Einbisskanal der Unterseite insgesamt 28 vertikale Stege und 10 kreisförmige Erhebungen aufweist.

In einer weiteren vorteilhaften Ausgestaltung der Aufbissschiene weist diese im Frontbereich ein schlitzförmiges Luftloch zur Sicherstellung einer Notatmung auf, welches die Aufbissschiene vollständig bis in den Mundhöhlenbereich durchsetzt, wobei das Luftloch länglich-oval oder länglich rechteckförmig gestaltet sein kann und vorzugsweise länglich-oval ist. In einer weiteren Ausgestaltung der Erfindung kann das schlitzförmige Luftloch wenigstens in den Eckzahnbereichen oder zwischen Eckzahnbereich und Schneidezahnbereich beginnen und über die Schneidezahnbereiche hinweglaufen. In einer besonderen Ausgestaltung kann das schlitzförmige Luftloch auch schon im Bereich des ersten Backenzahnes beginnen. Vorzugsweise weist dieser Luftschlitz eine Höhe von 1 bis 3 mm, insbesondere 2 mm und eine Länge von 10 bis 20 mm, vorzugsweise 12 bis 18 mm, insbesondere 15,8 mm auf.

In einer weiteren Ausgestaltung der Aufbissschiene beträgt die Dicke der Bissplatte zwischen dem Boden der Oberkieferschale und dem Boden der Unterkieferschale von 5 bis 8 mm, vorzugsweise 5,5 bis 7,5 mm, insbesondere 6 mm. Diese Dicke der horizontalen Bissplatte kann vorzugsweise durchgehend gleichbleibend außerhalb des Bereichs des Luftloches sein.

In einer weiteren Ausgestaltung der Aufbissschiene beträgt die die Dicke der Bissplatte 5 bis 8 mm, vorzugsweise 5,5 bis 7,5 mm, insbesondere 6 mm. Diese Dicke der horizontalen Bissplatte kann vorzugsweise durchgehend gleichbleibend außerhalb des Bereichs des Luftloches sein.

Aufgrund der Verdickung der horizontalen Bissplatte wird zum einen eine bessere, nämlich tiefere Kieferimpression, erreicht, zum anderen wird dadurch die Stabilität der Aufbissschiene ohne Verlust an Tragekomfort erhöht. Dadurch wird die Lebensdauer der Aufbissschiene, insbesondere bei Patienten mit nächtlichem Zähneknirschen, entscheidend verlängert.

In einer weiteren Ausgestaltung der Aufbissschiene weist dieselbe im Bereich um das schlitzförmige Luftloch eine Verdickung wenigstens der horizontalen Bissplatte der Oberkieferbissrille auf. Vorzugsweise weist auch die horizontale Bissplatte der Unterkieferbissrille um das schlitzförmige Luftloch eine eben solche Verdickung auf. Die Verdickung der Bissplatte im Bereich um das Luftloch herum gegenüber dem Bereich außerhalb des Luftloches beträgt von 1mm bis 4 mm, vorzugsweise von 1mm bis 2mm.

In einer weiteren Ausgestaltung der Erfindung beginnt die Verdickung der Bissplatte vor dem schlitzförmigen Luftloch im frontseitigen Kurvenbereich der Aufbissschiene, wenigstens im Eckzahnbereich, gegebenenfalls schon im Bereich des ersten Molaren, wobei die Verdickung in praktisch gleichbleibender Stärke über das Luftloch im Bereich der Schneidezähne hinweg läuft und zwar vorzugsweise sowohl im Bereich der Oberkieferbissrille als auch im Bereich der Unterkieferbissrille.

Um das Luftloch herum ist die horizontale Bissplatte auf der Unterseite bzw. auf der dem Unterkiefer zugewandten Seite um je zwischen 1mm bis 4mm, vorzugsweise zwischen 1mm bis 2mm verdickt, um im Frontbereich eine gute Impression der Schneidezähne zu erreichen.

In einer weiteren Ausgestaltung der Aufbissschiene ist die Geometrie der Front- und Seitenflächen zur Erhöhung des Tragekomforts abgeflacht, wobei die Abflachungen auf beiden Seiten zu den Enden der Aufbissschiene hin verlaufen. Die Front- und Seitenflächen werden durch Seitenwände gebildet, nämlich durch eine rechtsseitige innere und eine linksseitige sowie durch eine rechtsseitige und eine linksseitige Äußere Seitenwand. Die rechtsseitige innere bzw. Äußere Seitenwange geht im Bereich des Luftloches in die linksseitige innere bzw. Äußere Seitenwange über. In einer hierzu weiteren Ausgestaltung der Aufbissschiene ist die Bissleiste an ihrem linksseitigen wie rechtsseitigen molaren Ende verlängert bis unter den letzten Molaren, wobei im Bereich des letzten Backenzahnes die jeweiligen äußeren und inneren Seitenwangen auch geringfügig zurückgesetzt sein können. Die Aufbissschiene mitsamt Bissleiste reicht somit vorzugsweise so weit wie Backenzähne im Kiefer vorhanden sind. Durch diese Verlängerung der horizontalen Bissplatte nach hinten wird eine Auflage auch der hintersten Molaren in der oberen und unteren Zahnreihe erreicht, wodurch eine Entwicklung von theoretisch nicht auszuschließenden Backenzahnfehlstellungen bei langfristigem Gebrauch der Aufbissschiene sicher vermieden wird.

Die erfindungsgemäße Aufbissschiene besitzt den Vorteil, dass die Progenierung des Unterkiefers grundsätzlich durch eine gleichmäßige Impression sämtlicher Zähne erreicht wird. Des Weiteren besitzt die Aufbissschiene den Vorteil, dass sie unkompliziert ohne besondere Hilfsmittel innerhalb weniger Minuten angepasst werden kann. Aus diesem Grund genügt auch eine Standardschiene passend für fast sämtliche Kieferformationen. Des Weiteren ist in höchst vorteilhafter Weise eine individuelle Einstellung des Unterkiefervorschubs möglich.

Ausführungsbeispiele der Erfindung sind in den Zeichnungen dargestellt und werden im Folgenden näher beschrieben.

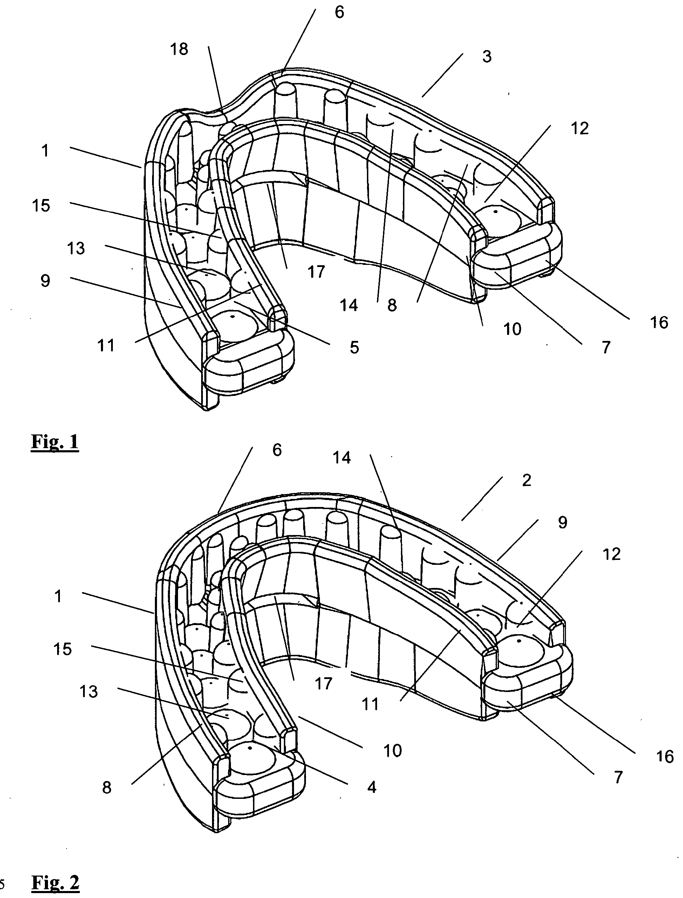

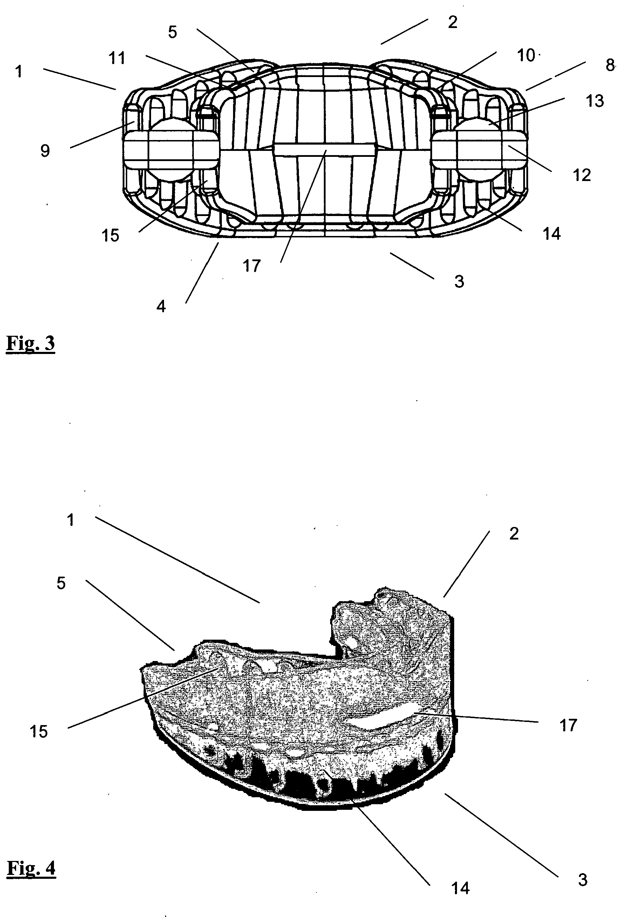

Figur 1 zeigt eine perspektivische Ansicht der Oberseite (3) einer Ausführungsform der erfindungsgemäßen Aufbissschiene (1) von hinten, Figur 2 zeigt die Unterseite (2) derselben erfindungsgemäßen Aufbissschiene (1) und Figur 3 zeigt sie von hinten.

Die Aufbissschiene (1) der Figuren 1, 2, und 3 besteht aus einem zahnspangenförmigen Körper, welcher entsprechend dem menschlichen Ober- und Unterkiefer gekrümmt ist. Die Aufbissschiene (1) enthält ein Material aus thermoplastischem Kunststoffgemisch, wobei die Aufbissschiene (1) dem menschlichen Ober- und Unterkiefer nachgeformt ist und in das Material der Aufbissschiene (1) im noch nicht angepassten Zustand zur Aufnahme der Zähne einen Einbisskanal für den Oberkiefer (5), Fig. 1, und einen Einbisskanal für den Unterkiefer (4), Fig. 2, eingeformt ist, wobei die Einbisskanäle eine Struktur aufweisen, welche die spätere Lage der Zähne vordefiniert.

Die Aufbissschiene (1) weist eine nach außen gewandte Frontseite (6) sowie eine nach innen, der Zunge zugewandte Rückseite (7) auf. Die Aufbissschiene (1) läuft nach hinten, das ist zum Inneren der Mundhöhle, abgeflacht aus und besitzt äußere bzw. bukkale Seitenflächen (8), (9), sowie innere bzw. linguale Seitenflächen (10), (11), welche in ihrer Dicke, Vertikalausdehnung, abnehmen, wie es den Fig. 1 und 2 zu entnehmen ist. Die Kanten der Seitenflächen (8), (9), (10), (11) sind vorzugsweise abgerundet, insbesondere im Bereich der hinteren Enden der Seitenwangen. Die Frontseite (6) (Fig.1) besitzt im oberen Bereich eine Einbuchtung (18) für den oberen Lippenbändchenbereich. In der Mitte wird die Aufbissschiene (1), ausgehend von der Frontseite (6) bis zur Rückseite, durch ein waagrecht verlaufendes, schlitzförmiges Luftloch (17) durchsetzt, welches somit die horizontal verlaufende Bissplatte (12), begrenzt durch den Boden der Oberkieferbissrille (5) und den Boden der Unterkieferbissrille (4), durchsetzt, wobei sich das schlitzförmige Luftloch (17) im Bereich der Schneidezähne befindet. Die horizontale Bissplatte (12) ist somit im Bereich des Luftloches (17) sowohl nach oben als auch nach unten verdickt.

Aus den Fig. 1, 2 und 3 ist deutlich zu erkennen, dass die horizontale Bissplatte (12) sowohl der Oberkieferbissrille (5) als auch der Unterkieferbissrille (4) mehrere Erhöhungen (13) aufweist. Die Oberkieferbissrille (5) (Fig. 1) weist jeweils im Bereich der Backenzähne vier solcher Erhöhungen (13) auf. Die Unterkieferbissrille (4) (Fig. 2) weist dagegen im Bereich der Backenzähne jeweils fünf solcher Erhöhungen (13) auf.

An den jeweiligen äußeren bzw. bukkalen Seitenwänden der Aufbissschiene (1) befinden sich an der Innenseite mehrere vertikale Verengungen (14). So umfasst die bukkale Seitenwand der Oberkieferbissrille (5) insgesamt elf solcher vertikaler Verengungen (14) (Fig. 1), davon eine im Bereich der Schneidezähne und jeweils fünf auf den beiden Seiten im Bereich der Backenzähne. Weiterhin umfasst die bukkale Seitenwand der Unterkieferbissrille (4) insgesamt fünfzehn solcher vertikaler Verengungen (14) (Fig.2), davon fünf im Bereich der Schneide- und Eckzähne und jeweils fünf auf den beiden Seiten im Bereich der Backenzähne.

An den jeweiligen inneren bzw. lingualen Seitenwänden der Aufbissschiene (1) befinden sich an der Innenseite mehrere vertikale Verengungen (15). So umfasst die linguale Seitenwand der Oberkieferbissrille (5) insgesamt elf solcher vertikaler Verengungen (15) (Fig. 2), davon eine im Bereich der Schneidezähne und jeweils fünf auf den beiden Seiten im Bereich der Backenzähne. Weiterhin umfasst die linguale Seitenwand der Unterkieferbissrille (4) insgesamt dreizehn solcher vertikaler Verengungen, davon einen im Bereich der Schneidezähne und jeweils sechs auf den beiden Seiten im Bereich der Eck- und Backenzähne.

Ein weiterer Vorteil der erfindungsgemäßen Aufbissschiene 1 besteht darin, dass diese in weitaus den meisten Fällen keine Anpassung durch Schneiden von Material der Seitenflächen 8, 9, 10 und 11 oder der Frontfläche 6 benötigt. Die Aufbissschiene 1 ist als Standardschiene ausgeführt, welche für fast sämtliche Kieferformationen passt und deshalb in aller Regel keinen individuellen Zuschnitt benötigt. Auf Grund der durch die Erhöhungen (13) der Bissplatte (12) und den vertikalen Verengungen (14), (15) der bukkalen und der lingualen Seitenwände vorgegebenen Strukturen in den jeweiligen Bissrillen erfolgt eine sichere, stabile Positionierung der Zähne des Anwenders beim Aufbiss, was zu einer längeren Tragestabilität und höherer Compliance führt.

## Patentansprüche

1. Aufbissschiene (1) aus thermoplastischen Material zur Verhinderung von Schnarchen und von obstruktiver Schlafapnoe, welche es erlaubt, den Unterkiefer gegenüber dem Oberkiefer in eine leicht nach vorne gelagerte Position zu verschieben umfassend eine Unter- (2) und eine Oberseite (3), die jeweils einen zum Unter- bzw. Oberkiefer geöffneten, Formgebenden Einbisskanal (4, 5) für die Zähne bilden, wobei das thermoplastische Material zahnspangenartig dem menschlichen Ober- und Unterkiefer nachformbar ist, wobei die innere Oberfläche der Einbisskanäle eine Struktur aufweist, welche die Lage der Zähne in den Einbisskanälen definiert und deren Haftung in dem thermoplastischen Material verbessert, **dadurch gekennzeichnet, dass** der innere Boden (12) der beiden Einbisskanäle im Bereich der Eck- und Backenzähne eine oder mehrere flache Erhöhungen (13) mit einem im Wesentlichen kreisförmigen Bodenumfang aufweist und die inneren Seitenwände der beiden Einbisskanäle eine oder mehrere Verengungen in Form von vertikalen Stegen (14, 15) aufweisen.

2. Aufbissschiene (1) nach Anspruch 1, **dadurch gekennzeichnet, dass** die beiden Einbisskanäle (4, 5) durch vertikale Stege (14, 15) jeweils auf beiden Seiten im Bereich der Backenzähne verengt werden.

3. Aufbissschiene nach einem der Ansprüche 1 oder 2, **dadurch gekennzeichnet, dass** die inneren Seitenwände der beiden Einbisskanäle (4, 5) jeweils auf beiden Seiten im Bereich der Eck und Schneidezähne Verengungen (14, 15) aufweisen.

4. Aufbissschiene nach einem der Ansprüche 1 bis 3, **dadurch gekennzeichnet, dass** jede der vorderen flachen Erhöhungen (13) des inneren Bodens (12) von Verengungen (14, 15) der inneren Seitenwände flankiert ist.

5. Aufbissschiene nach einem der Ansprüche 1 bis 4, **dadurch gekennzeichnet, dass** die Verengungen (14, 15) der inneren Seitenwände im getragen Zustand jeweils in den Zwischenzahnräumen liegen und die flachen Erhöhungen (13) des inneren Bodens (12) jeweils im Bereich der Zahnkronen liegen.

6. Aufbissschiene nach einem der Ansprüche 1 bis 5, **dadurch gekennzeichnet, dass** der Boden im hinteren Bereich der Einbisskanäle (4, 5) über die Seitenwände hinaus zur Unterstützung der letzten Backenzähne nach hinten verlängert ist.

7. Aufbissschiene nach einem der Ansprüche 1 bis 6, **dadurch gekennzeichnet, dass** das thermoplastisches Material ein Material ist, das sich in der Wärme, unterhalb von 60 °C, plastisch verformen lässt und sich eng an eine vorgegebene Form anschmiegt und danach beim Abkühlen diese besagte Form beibehält.

8. Aufbissschiene nach einem der Ansprüche 1 bis 7, **dadurch gekennzeichnet, dass** der innere Boden (12) der beiden Einbisskanäle (4, 5) jeweils 3 bis 12 flache Erhöhungen (13) mit im Wesentlichen kreisförmigen Bodenumfang aufweist.

9. Aufbissschiene nach einem der Ansprüche 1 bis 8, **dadurch gekennzeichnet, dass** der innere Boden (12) des oberen Einbisskanals (5) auf beiden Seiten jeweils im Bereich der Backenzähne 4 flache Erhöhungen (13) aufweist und der innere Boden (12) des unteren Einbisskanals (4) auf beiden Seiten jeweils im Bereich der Backenzähne 5 flache Erhöhungen (13) aufweist.

## Claims

1. A bite splint (1) of a thermoplastic material to prevent snoring and obstructive sleep apnea, which bite splint allows moving the lower jaw relative to the upper jaw into a slightly forward shifted position, comprising a lower (2) and an upper side (3), each of which defining a shaping bite channel (4, 5) that is open towards the lower or upper jaw, respectively, wherein the thermoplastic material can be molded to model the human lower and upper jaw in a brace-like manner, wherein the inner surface of the bite channels comprises a structure which improves the position of the teeth in the bite channels and their adhesion in the thermoplastic material,
**characterized in that** the inner bottom (12) of the two bite channels comprises one or more flat raised areas (13) with a substantially circular bottom perimeter in the region of the canines and molars and **in that** the inner side walls of the two bite channels comprise one or more narrowed sections in the form of vertical webs (14, 15).

2. The bite splint (1) according to claim 1, **characterized in that** the two bite channels (4, 5) are narrowed on both sides by vertical webs (14, 15) in the region of the molars.

3. The bite splint according to either one of claims 1 or 2, **characterized in that** the inner side walls of the two bite channels (4, 5) each comprise narrowed sections (14, 15) on both sides in the region of the canines and molars.

4. The bite splint according to any one of claims 1 to 3, **characterized in that** each of the front flat raised areas (13) of the inner bottom (12) is flanked by narrowed sections (14, 15) of the side walls.

5. The bite splint according to any one of claims 1 to 4, **characterized in that** the narrowed sections (14, 15) of the inner side walls are located in the interdental spaces and the flat raised areas (13) of the inner bottom (12) are located in the region of the dental crowns when the bite splint is worn.

6. The bite splint according to any one of claims 1 to 5, **characterized in that** the bottom in the rear section of the bite channels (4, 5) is extended backwards beyond the side walls to support the last molars.

7. The bite splint according to any one of claims 1 to 6, **characterized in that** the thermoplastic material is a material that can be plastically deformed and conforms closely to a defined form in heat below 60°C, and will then this said form during cooling.

8. The bite splint according to any one of claims 1 to 7, **characterized in that** the inner bottom (12) of the two bite channels (4, 5) comprises 3 to 12 flat raised areas (13) with a substantially circular bottom perimeter.

9. The bite splint according to any one of claims 1 to 8, **characterized in that** the inner bottom (12) of the upper bite channel (5) comprises 4 flat raised areas (13) on both sides in the region of the molars and the inner bottom (12) of the lower bite channel (4) comprises 5 flat raised areas (13) on both sides in the region of the molars.

## Revendications

1. Appareil dentaire (1) fabriqué à partir d'un matériau thermoplastique pour éviter les ronflements et l'apnée du sommeil obstructive, lequel permet de déplacer la mâchoire inférieure par rapport à la mâchoire supérieure dans une position légèrement disposée vers l'avant comprenant un côté inférieur (2) et un côté supérieur (3), qui forment respectivement un canal de mastication (4, 5) de façonnage pour les dents qui est ouvert vers la mâchoire inférieure ou la mâchoire supérieure, le matériau thermoplastique pouvant reproduire la mâchoire supérieure et la mâchoire inférieure de l'être humain sous forme de bagues, la surface interne des canaux de mastication présentant une structure, laquelle définit la position des dents dans les canaux de mastication et qui améliore leur adhérence dans le matériau thermoplastique, **caractérisé en ce que** le fond intérieur (12) des deux canaux de mastication présente dans la zone des canines et des molaires une ou plusieurs protubérances plates (13) avec un périmètre de fond sensiblement de forme circulaire et les parois latérales des deux canaux de mastication présentent un ou plusieurs rétrécissements sous forme de barres verticales (14, 15).

2. Appareil dentaire (1) selon la revendication 1, **caractérisé en ce que** les deux canaux de mastication (4, 5) ont été rétrécis par des barres verticales (14, 15) respectivement des deux côtés dans la zone des molaires.

3. Appareil dentaire selon l'une quelconque des revendications 1 ou 2, **caractérisé en ce que** les parois latérales internes des deux canaux de mastication (4, 5) présentent des rétrécissements (14, 15) respectivement des deux côtés dans la zone des canines et des molaires.

4. Appareil dentaire selon l'une quelconque des revendications 1 à 3, **caractérisé en ce que** chacune des protubérances plates (13) frontales du fond intérieur (12) est assortie de rétrécissements (14, 15) des parois latérales internes.

5. Appareil dentaire selon l'une quelconque des revendications 1 à 4, **caractérisé en ce que** les rétrécissements (14, 15) des parois latérales internes à l'état porté se trouvent respectivement dans les espaces interdentaires et les protubérances plates (13) du fonds intérieur (12) se trouvent respectivement dans la zone des couronnes dentaires.

6. Appareil dentaire selon l'une quelconque des revendications 1 à 5, **caractérisé en ce que** le fond dans la zone arrière des canaux de mastication (4, 5) est prolongé au-delà des parois latérales pour soutenir les dernières molaires vers l'arrière.

7. Appareil dentaire selon l'une quelconque des revendications 1 à 6, **caractérisé en ce que** le matériau thermoplastique est un matériau dont le plastique est déformable sous l'effet de la chaleur à une température inférieure à 60 °C et qui épouse étroitement une forme prédéfinie, et qui conserve ensuite lors du refroidissement ladite forme.

8. Appareil dentaire selon l'une quelconque des revendications 1 à 7, **caractérisé en ce que** le fond intérieur (12) des deux canaux de mastication (4, 5) présente respectivement 3 à 12 protubérances plates (13) avec un périmètre de fond sensiblement de forme circulaire.

9. Appareil dentaire selon l'une quelconque des revendications 1 à 8, **caractérisé en ce que** le fond intérieur (12) du canal de mastication supérieur (5) présente 4 protubérances plates (13) des deux côtés respectivement dans la zone des molaires, et le fond intérieur (12) du canal de mastication inférieur (4) présente 5 protubérances plates (13) des deux côtés respectivement dans la zone des molaires.
